# EUROPÄISCHE PATENTSCHRIFT

(11) **EP 4 260 557 B1**
(45) Veröffentlichungstag und Bekanntmachung des Hinweises auf die Patenterteilung: **28.01.2026**
(21) Anmeldenummer: 21831272.6
(22) Anmeldetag: 07.12.2021
(51) Int. Cl.: H04N 17/00, A61B 1/00, A61B 1/06, G06T 7/90, H04N 9/73, H04N 23/50, H04N 23/88

(54) **VERFAHREN ZUM KALIBRIEREN EINER MEDIZINISCHEN BILDGEBUNGSVORRICHTUNG SOWIE MEDIZINISCHE BILDGEBUNGSVORRICHTUNG**
METHOD FOR CALIBRATING A MEDICAL IMAGING DEVICE AND MEDICAL IMAGING DEVICE
PROCÉDÉ POUR ÉTALONNER UN DISPOSITIF D'IMAGERIE MÉDICALE ET DISPOSITIF D'IMAGERIE MÉDICALE

(30) Priorität: 08.12.2020 DE 102020132564; 09.04.2021 DE 102021108932
(43) Veröffentlichungstag der Anmeldung: 18.10.2023
(73) Patentinhaber: Karl Storz SE & Co. KG, 78532 Tuttlingen (DE)
(72) Erfinder: BUSCHLE, Lukas, 78532 Tuttlingen (DE); FALLERT, Johannes, 78532 Tuttlingen (DE); GÖBEL, Werner, 78532 Tuttlingen (DE)
(86) Internationale Anmeldenummer: PCT/EP2021/084577
(87) Internationale Veröffentlichungsnummer: WO 2022/122725

(56) Entgegenhaltungen:
- JP-A- 2009 273 676
- JP-A- 2013 090 884
- JP-A- 2015 134 110
- US-A1- 2018 299 552
- US-A1- 2019 129 037

## Beschreibung

Die Erfindung betrifft ein Verfahren zum Kalibrieren einer medizinischen Bildgebungsvorrichtung, insbesondere eines Endoskop-Systems oder eines Exoskop-Systems, wobei die Bildgebungsvorrichtung ein optisches System mit einer Optik und einem Bildsensor zum Aufnehmen eines Bildes eines Betrachtungsbereichs aufweist und eine Bildinformation des Bildes durch einen inneren und/oder äußeren Einfluss, insbesondere das optische System, eine Beleuchtung und/oder eine Umgebungsbedingung, einer Spektralabweichung unterliegt, mit mehreren Schritten. Weiterhin betrifft die Erfindung eine medizinische Bildgebungsvorrichtung, insbesondere ein medizinisches Endoskop oder ein medizinisches Exoskop.

Bekannte medizinische Bildgebungsvorrichtungen, wie beispielsweise Endoskope oder Exoskope, werden sowohl zum Aufnehmen von Bildern unter beschränkten geometrischen Gegebenheiten als auch zum Analysieren bestimmter Parameter in einem Betrachtungsbereich verwendet. Dazu sind unter anderem sogenannte hyperspektrale oder auch multispektrale Imaging-Systeme (HSI/MSI) für Endoskope oder für Exoskope bekannt.

Weiterhin sind diverse Endoskope bekannt, bei welchen eine entsprechende Optik an der Spitze des jeweiligen Endoskopes drehbar ausgestaltet ist. Diese können auch mit HSI und/oder MSI-Systemen kombiniert sein.

Zudem ist es üblich, auf Endoskope oder Exoskope mit einem einfachen Okular für das Betrachten mit dem menschlichen Auge eine Kamera auf das Okular am Kopf des jeweiligen Gerätes aufzusetzen, sodass ein externes Anzeigen des Bildes eines Betrachtungsbereiches ermöglicht ist.

Bekannten Endoskopen oder auch Exoskopen ist gemein, dass bei einem Verändern eines Abstandes zu einem Betrachtungsbereich oder zu einem betrachteten Gegenstand, wie beispielsweise einem Organ und/oder auch durch das Drehen eines optischen Systems oder einer Optik oder beispielsweise einer aufgesetzten Kamera eine für ein hyperspektrales oder multispektrales Imaging-System notwendige Kalibrierung nur für eine Grundstellung vorliegt, und daher bei einem Durchführen der beschriebenen Veränderungen eine Veränderung der Bildqualität in Bezug auf die ermittelten Ergebnisse erzeugt ist, da die Kalibrierung verloren geht. Verwandter Stand der Technik findet sich weiterhin in den Druckschriften JP2013090884 A, JP2009273676 A, US2019/129037 A1, JP2015134110 A, US2018/299552 A1.

Aufgabe der Erfindung ist es, den Stand der Technik zu verbessern.

Gelöst wird die Aufgabe durch ein Verfahren zum Kalibrieren einer medizinischen Bildgebungsvorrichtung, insbesondere eines Endoskop-Systems oder eines Exoskop-Systems, nach Anspruch 1.

Durch das Bestimmen einer Spektralverteilung der in einer realen Einsatzumgebung bestimmten ersten Aufnahme kann eine entsprechende Spektralabweichung von einer Referenzspektralverteilung ermittelt werden. So liegt für jede erste Aufnahme eine Information darüber vor, inwieweit eine solche Spektralverteilung der ersten Aufnahme von einer Referenzspektralverteilung abweicht. Durch diese bestimmte Spektralabweichung kann dann ein Korrigieren der Bildinformation auf Basis dieser Abweichung von der Referenzspektralverteilung erfolgen, sodass eine korrigierte Bildinformation vorliegt. Die korrigierte Bildinformation ist somit um die Spektralabweichung kompensiert oder auf die jeweilige Einsatzsituation mit veränderten Parametern neu kalibriert, sodass eine reduzierte oder eliminierte Spektralabweichung vorliegt. Damit kann in einer jeweiligen Einsatzumgebung ein genaues Bestimmen von beispielsweise Gewebeparametern mittels beispielsweise eines MSI erfolgen, da das Bestimmen dieser Gewebeparameter von einer entsprechend genauen Ermittlung entsprechender Spektralverteilungen abhängt.

Folgende Begriffe seien an dieser Stelle erläutert:
Ein "Kalibrieren" beschreibt in der Messtechnik oder für einen Messprozess eine Feststellung und/oder Dokumentation einer Abweichung des jeweiligen Messgerätes gegenüber einem anderen Messgerät oder einem Referenzmaß. Das Referenzmaß wird in diesem Fall auch als "Normal" bezeichnet. Ein solches Kalibrieren oder eine solche Kalibrierung umfasst weiterhin einen zweiten Schritt, nämlich ein Berücksichtigen der ermittelten Abweichung bei einem anschließenden Benutzen des jeweiligen Messgerätes zum Korrigieren der abgelesenen Werte. Eine sogenannte kalibrierte Messung liefert damit genauere Ergebnisse oder sogar exakte Ergebnisse innerhalb einer angestrebten minimalen Abweichung von der Realität gegenüber einer nicht kalibrierten Messung.

Eine "medizinische Bildgebungsvorrichtung" ist beispielsweise ein Endoskop, ein Endoskop-System oder auch ein Exoskop oder Exoskop-System. Weiterhin kann eine solche medizinische Bildgebungsvorrichtung jede Einrichtung sein, welche geeignet ist, mit einem Bildgebungsverfahren in einem medizinischen Umfeld eingesetzt zu werden, beispielsweise zum Ermitteln eines entsprechenden Bildes oder auch zum Bestimmen von Parametern eines betrachteten Bereichs. Ein Endoskop ist dabei ein Gerät, mit dem beispielsweise ein Innenbereich eines Menschen, beispielsweise ein Bauchraum, untersucht und Teile darin manipuliert werden können. Ein solches Endoskop umfasst dabei beispielsweise sowohl optische Komponenten als auch mechanische Komponenten zum Manipulieren eines entsprechend betrachteten Bereichs oder Teilen in diesem Bereich. Hierzu wird korrekt von einem Endoskop-System gesprochen, wobei das "Endoskop" die eigentlichen Teile zur Bildaufnahme bezeichnet. Häufig werden diese Begriffe jedoch auch synonym verwendet. Ein Exoskop oder auch ein Exoskop-System ist dabei ein vergleichbares Instrument zum Betrachten und/oder Manipulieren von offenliegenden Bereichen eines Organismus, beispielsweise eines Menschen während eines offenen Eingriffs. Ein Bestimmen von Gewebeparametern oder anderen Parametern erfolgt dabei beispielsweise mittels einer Spektralanalyse, häufig unter Berücksichtigung von entsprechend gezielt eingesetzten Beleuchtungseinrichtungen spezieller Spektren. So kann beispielsweise mittels einer solchen medizinischen Bildgebungsvorrichtung eine Analyse per Hyperspektral-Imaging (HSI) oder Multispektral-Imaging (MSI) erfolgen.

Ein "optisches System" kann dabei jede Einrichtung der medizinischen Bildgebungsvorrichtung sein, welche dazu geeignet oder dazu eingerichtet ist, Bildinformationen oder andere optische Informationen aufzunehmen, weiterzuleiten und/oder abzugeben. Ein solches optisches System führt dabei beispielsweise Licht aus dem Betrachtungsbereich bis zu einem Bildsensor oder Bildaufnahmechip.

Eine "Optik" beschreibt dabei die Gesamtheit von optisch leitenden Komponenten. Beispielsweise gehören zu einer solchen Optik Linsen, Lichtleiter oder entsprechende Filter, aber auch auf eine Endoskop-System zusätzlich aufgesetzte optische Komponenten.

Ein "Bildsensor" ist beispielsweise eine Vorrichtung oder auch Einrichtung zum Aufnehmen von lichtbasierten Informationen auf elektrischem Wege. Beispielsweise ist ein solcher Bildsensor ein halbleiterbasiertes Bauteil, welches sichtbares sowie auch nicht sichtbares Licht aufnehmen kann. Allgemein kann ein solcher Bildsensor jede Art von elektromagnetischer Strahlung aufnehmen, wenn dieser für einen entsprechenden Wellenlängenbereich eingerichtet ist. Ein solcher Bildsensor ist dabei beispielsweise an der Spitze, in einem Griff oder einem Kopf eines Endoskops oder eines Exoskops oder auch in einer externen, auf das Endoskop-System oder Exoskop-System aufgesetzten Kamera angeordnet sein.

Ein "Bild" bezeichnet ein Abbild oder eine Darstellung eines entsprechenden Betrachtungsbereiches, beispielsweise als Farbpixel oder Gesamtheit von Farbpixeln oder eine dazu jeweils äquivalente elektronische Funktion. Ein solches Bild muss dabei nicht physisch oder für einen Menschen sichtbar vorliegen, sondern kann während eines Verarbeitens oder eines Speicherns oder eines Vorbereitens für eine Darstellung für einen Menschen auch in elektronischer Form vorliegen und die Informationen eines entsprechenden Bildes eines Betrachtungsbereiches umfassen.

Ein "Betrachtungsbereich" ist insbesondere der Bereich, welcher mit der medizinischen Bildgebungsvorrichtung betrachtet wird, beispielsweise ist ein solcher Betrachtungsbereich ein Bauchraum, ein Organ oder ein anderer Bestandteil eines Organismus.

Eine "Bildinformation" ist dabei der Aussagegehalt des beschriebenen Bildes, beispielsweise also eine entsprechende Anzahl von Pixeln in entsprechender Färbung, eine Information über eine entsprechende Spektralverteilung des jeweiligen Bildes je Pixel oder auch eine andere, beispielsweise Meta-Information, des jeweiligen Bildes.

Ein "innerer und/oder äußerer Einfluss" beschreibt jeden Einfluss auf die Bildinformation, welcher die Qualität der Bildinformation oder einen Aussagegehalt der Bildinformation verändert oder verschlechtert und welcher insbesondere durch das optische System, durch eine Beleuchtung und/oder durch eine Umgebungsbedingung entsteht oder verursacht wird. Ein solcher Einfluss durch das optische System kann beispielsweise eine Asymmetrie einer entsprechenden Linse, eine über eine Gesamtfläche ungleiche Filterwirkung eines Filters oder eines optischen Bestandteils oder auch im einfachsten Falle eine Verschmutzung im optischen System sein. Ein entsprechender Einfluss durch eine Beleuchtung entsteht beispielsweise durch eine ungleiche Spektralverteilung einer entsprechenden Lichtquelle, welche zur Beleuchtung dient. Eine Umgebungsbedingung kann dabei beispielsweise ein entsprechender Hintergrund um den Betrachtungsbereich sein, welcher reflektiertes oder gestreutes Licht entsprechend verfälscht, beispielsweise mit einem anderen Farbspektrum überlagert, oder reflektiert oder absorbiert, sodass eine Beleuchtungsintensität verändert wird.

Eine "Beleuchtung" ist in diesem Zusammenhang insbesondere eine gezielte, multispektrale und/oder mit definiertem Lichtspektrum durchgeführte Beleuchtung des Betrachtungsbereiches, welche dann beispielsweise für ein MSI- oder HSI-imaging verwendet wird. Dabei kann auch eine Spektralabweichung durch diese entsprechende Beleuchtung verursacht sein, da auch in den entsprechend von der Beleuchtung ausgesandten Lichtspektren Abweichungen zum angestrebten Spektrum vorkommen können.

Eine "Spektralabweichung" kann gewollt oder ungewollt entstehen und ist beispielsweise ein ungewolltes Filtern eines entsprechenden Lichtspektrums, ein Abschatten bestimmter Spektralbereiche oder ein Beeinflussen einer entsprechenden Spektralverteilung in ihrer Intensität in entsprechenden Spektralbereichen. So kann eine gewollte Spektralabweichung beispielsweise auch dadurch erzeugt sein, dass mit einer entsprechenden Beleuchtung beleuchtet wird, um anhand eines dann der Bildinformation zugeführten Lichtspektrums eine Analyse von Gewebeparametern vorzusehen. Eine solche Spektralabweichung kann dabei insbesondere inhomogen über mehrere Pixel oder über alle Pixel des Bildes oder auch über das ganze Bild inhomogen verteilt sein.

Ein "Aufnehmen" einer Bildinformation beschreibt beispielsweise das optische Zuführen der entsprechenden Bildinformation oder eines dazu korrespondierenden Bildes in Richtung des Bildsensors sowie das Umwandeln der Bildinformation auf dem Bildsensor in beispielsweise ein elektronisches Signal.

Eine "Aufnahme" beschreibt dabei ein konkretes, identifizierbares und zuzuordnendes Bild des entsprechenden Bildbereiches im Betrachtungsbereich.

"Bestimmen" einer Spektralverteilung beschreibt beispielsweise das Erzeugen einer Information derart, dass eine entsprechende Intensität des in der Aufnahme vorliegenden jeweiligen Lichtes bestimmten Spektren zugeordnet oder zu bestimmten Frequenzen des Lichts zugeordnet werden, sodass eine nachvollziehbare Information über eine entsprechende Spektralverteilung vorliegt.

Ein "Ermitteln" einer Spektralabweichung beschreibt ein Vergleichen der Spektralverteilung mit einer jeweiligen Aufnahme mit einer Referenzspektralverteilung, sodass beispielsweise mittels einer Differenzbildung zu entsprechenden Frequenzen eine jeweilige Information über eine Abweichung in der Intensität des jeweils zu der Frequenz eintreffenden Lichtes vorliegt.

Eine "Referenzspektralverteilung" beschreibt dabei beispielsweise eine während der Herstellung oder einer Qualitätssicherung für eine entsprechende medizinische Bildgebungsvorrichtung festgestellte, der jeweiligen medizinischen Bildgebungsvorrichtung zugeordneten Spektralverteilung, welche eine möglichst reale Abbildung einer Bildinformation des Betrachtungsbereiches ermöglicht. Eine solche Referenzspektralverteilung dient damit beispielsweise als "Normal" im Sinne einer angestrebten Kalibrierung. Eine solche Referenzspektralverteilung ist dabei insbesondere inhomogen und/oder definiert inhomogen über das Bild verteilt und ermöglicht eine Kalibrierung entsprechen der daraus entstehenden Inhomogenität. Damit kann, beispielsweise im Unterschied zu einem Weißabgleich, eine pixelgenaue Kalibrierung erfolgen.

Ein "Korrigieren" der Bildinformation beschreibt ein rechnerisches Beeinflussen der Bildinformation derart, dass mittels der Spektralabweichung die Bildinformation derart korrigiert wird, dass die Bildinformation korrespondierend zur Referenzspektralverteilung der medizinischen Bildgebungsvorrichtung vorliegt. Ein Ergebnis eines solchen Korrigierens ist dann beispielsweise die "korrigierte Bildinformation".

Um das Verfahren vorteilhaft in hochauflösenden Bildgebungsverfahren und für übliche Bildinformationen durchführen zu können, wird das Aufnehmen einer Bildinformation, das bestimmen einer Spektralverteilung, das Ermitteln einer Spektralabweichung und/oder das Korrigieren der Bildinformation für ein Pixel oder für ein jeweiliges Pixel des Bildes und/oder der Bildinformation durchgeführt.

Im Ergebnis kann für ein jeweiliges Pixel oder beispielsweise auch für alle Pixel eines entsprechenden Bildes oder einer entsprechenden Bildinformation eine kalibrierte Spektralverteilung erzeugt werden, sodass auch in hochauflösenden Bildern mit einer Vielzahl von Pixeln ein sehr genaues Bestimmen beispielsweise einer Gewebeeigenschaft mittels einer Multispektralanalyse möglich ist.

Ein "Pixel", welches auch Bildpunkt, Bildzelle oder Bildsegment bezeichnet wird, bezeichnet einzelne Farbwerte oder auch Helligkeitswerte einer digitalen Rastergraphik, wie sie üblicherweise in elektronischen Bildgebungsverfahren genutzt werden. Ein solches Pixel bezeichnet dabei auch ein entsprechendes Flächenelement, beispielsweise eines Bildsensors, oder auch eines Bildschirms, wobei eine Vielzahl von Pixeln dann ein vollständiges Bild ergibt.

In einer Ausführungsform erfolgt das Aufnehmen einer Bildinformation, das Bestimmen einer Spektralverteilung, das Ermitteln einer Spektralabweichung und/oder das Korrigieren der Bildinformation für einen Spektralbereich oder mehrere Spektralbereiche.

Mit diesem Vorgehen wird es ermöglicht, ein Kalibrieren möglichst exakt für den jeweiligen Spektralbereich vorzunehmen, sodass beispielsweise ein Kalibrieren abgestimmt auf ein multispektrales Analyseverfahren zum Bestimmen einer Gewebeeigenschaft erfolgen kann.

Ein "Spektralbereich" beschreibt dabei beispielsweise einen Ausschnitt aus einem Gesamtspektrum einer entsprechenden Spektralverteilung.

Dazu erfolgt dann in einer Ausführungsform ein Korrigieren der Bildinformation auf Basis der Abweichung von der Referenzspektralverteilung für einen Spektralbereich oder mehrere Spektralbereiche, sodass die Bildinformation derart aufbereitet ist, dass die korrigierte Bildinformation mit einer reduzierten oder eliminierten Spektralabweichung für einen jeweiligen Spektralbereich oder mehrere jeweilige Spektralbereiche vorliegt.

Erfindungsgemäss wird ein Abstand vom Betrachtungsbereich ermittelt und die Referenzspektralverteilung abhängig von diesem Abstand des Betrachtungsbereichs bestimmt, wobei das Korrigieren der Bildinformation anhand des ermittelten Abstands vom Betrachtungsbereich durchgeführt wird.

Mittels dieses Abstands kann damit ein Kalibrieren bezüglich abstandsabhängiger Spektralabweichungen erfolgen. So führt ein Abstand zum Betrachtungsbereich beispielsweise dazu, dass Reflexionen von einem Hintergrund des Betrachtungsbereiches zu einer Spektralabweichung führen.

Um diesen Abstand vom Betrachtungsbereich zuverlässig zu ermitteln, wird der Abstand mittels eines Lasers, eines Ultraschallsystems und/oder mittels einer Bildauswertung bestimmt.

So kann beispielsweise ein Laser-Interferometer, ein Laserentfernungsmesser oder eine Einrichtung zum Messen einer Laufzeit eines Lasers, auch "Time-of-Flight"-Analyse genannt, dazu genutzt werden, den Abstand zuverlässig und präzise zu ermitteln. Ein Ultraschallsystem kann beispielsweise über eine Echolaufzeit einen entsprechenden Abstand ermitteln. Weiterhin ist es möglich, dem Abstand mittels einer Bildauswertung zu bestimmen. Dazu erfolgt eine Korrelation oder auch eine Triangulation entsprechender Bildinformationen, beispielsweise in einem Stereo-Endoskop, sodass im Ergebnis auf den Abstand geschlossen werden kann.

Erfindungsgemäss wird das Korrigieren der Bildinformation anhand einer ermittelten rotatorischen Position des optischen Systems und/oder der Optik durchgeführt.

Somit kann beispielsweise bei einem Endoskop oder auch Exoskop mit drehbarer Spitze oder mit einer drehbaren, auf einem Okular aufgesetzten Kamera ein entsprechendes Korrigieren der Bildinformation abhängig von der rotatorischen Position erfolgen. Folglich kann zu jeder rotatorischen Position auch eine entsprechende Kalibrierung vorgenommen werden, welche beispielsweise auch laufend und/oder pixelgenau erfolgt. Dazu wird zu jeder inkrementellen rotatorischen Position ein Korrigieren gegenüber einer Referenzspektralverteilung durchgeführt.

Eine "rotatorische Position" beschreibt dabei beispielsweise eine entsprechende Winkelangabe gegenüber einem Null-Winkel oder gegenüber einer Ausgangsstellung des optischen Systems und/oder der Optik. So kann beispielsweise die rotatorische Position als Winkelangabe in "°" angegeben sein. Zu jeder entsprechenden rotatorischen Position oder zu entsprechenden Inkrementen rotatorischer Positionen im Bezug zu einem Vollkreis kann dann eine entsprechende Korrekturinformation in Form einer Referenzspektralverteilung vorliegen. Insbesondere ist damit eine rotatorische Position des Bildsensors in Bezug zum optischen System und/oder in Bezug zum Betrachtungsbereich bezeichnet.

Um die rotatorische Position zuverlässig ohne weitere Bauteile ermitteln zu können, wird die rotatorische Position anhand eines dem optischen System und/oder der Optik zugeordneten optischen Indikators für die rotatorische Position ermittelt.

So kann beispielsweise eine Bildauswertung derart erfolgen, dass eine einer Winkelposition zuzuordnende Anomalie oder geometrische Abweichung eines entsprechenden Bildes dazu genutzt wird, die rotatorische Position festzustellen.

Ein "optischer Indikator" kann dabei beispielsweise ein Ausschnitt einer ansonsten kreisförmigen Bildfläche, eine Markierung, ein Punkt oder ein anderes geometrisches Gebilde im optischen System und/oder in der Optik sein, welches vom Bildsensor und/oder der daran angeschlossenen Komponenten wie beispielsweise eines Anzeigeinstrumentes mit Auswertetechnik, erkannt werden kann. Weiterhin kann als optischer Indikator auch eine in einer Fertigung einer entsprechenden medizinischen Bildgebungsvorrichtung erzeugte Abweichung oder Qualitätsabweichung genutzt werden. Alternativ oder ergänzend kann ein optischer Indikator auch durch Referenzbildpunkte einer Bildauswertung erzeugt werden, sofern diese im Bild Abhängig von der Rotation vorliegen.

Alternativ oder auch zum Erzeugen einer Redundanz wird die rotatorische Position anhand eines Sensors ermittelt.

Ein "Sensor", welcher auch "Messfühler" genannt wird, ist ein technisches Bauteil, welches physikalische oder chemische Eigenschaften und/oder auch stoffliche Eigenschaften oder Beschaffenheiten in seiner jeweiligen Umgebung qualitativ und/oder quantitativ erfassen kann. Beispielsweise wird dann ein entsprechendes elektronisches Signal erzeugt, welches weitergeleitet und/oder verarbeitet werden kann.

Die rotatorische Position kann dabei anhand eines Magnet-Sensors, eines Hall-Sensors, eines Laser-Sensors, eines Licht-Sensors und/oder eines Inkrementgebers ermittelt werden.

Ein "Magnet-Sensor" kann dabei jeder Sensor sein, welcher magnetische Eigenschaften zum Detektieren der rotatorischen Position nutzt. Beispielsweise umfasst ein solcher Magnet-Sensor einen magnetisierten Ring, welcher von einem Magnet-Aufnehmer abgetastet wird und damit eine rotatorische Position ermittelt.

Ein solcher Magnet-Sensor kann auch als "Hall-Sensor" ausgeführt sein, welcher mittels des sogenannten Hall-Effekts Magnetfelder bestimmen kann. So kann ein entsprechender magnetischer Bestandteil des optischen Systems und/oder der Optik gegenüber einem entsprechenden Hall-Sensor positioniert sein und ein Verändern der rotatorischen Position des optischen Systems und/oder der Optik zu einer Signalveränderung am Hall-Sensor führen.

Ein "Laser-Sensor" kann jeder Sensor sein, welcher mittels Laserstrahl oder der Nutzung eines Laserstrahls ein Messsignal erzeugt. Beispielsweise kann ein solcher Laser-Sensor eine umlaufende optische Markierung am optischen System und/oder an der Optik abtasten, sodass ein Signal bezüglich der rotatorischen Position erzeugt wird.

Entsprechend eine rotatorische Position auch mittels eines Lichtsensors abgetastet werden, wobei beispielsweise ein Bild eines Inkrement-Ringes oder eines Inkrementgebers ausgelesen und entsprechend ausgewertet wird.

Um mittels der medizinischen Bildgebungsvorrichtung Gewebeeigenschaften zuverlässig und präzise auswerten zu können, wird die korrigierte Bildinformation für ein HSI oder ein MSI ausgewertet. Dies erfolgt insbesondere zum Bestimmen von Eigenschaften des Betrachtungsbereichs, insbesondere eines Hämoglobingehalts, eines Wassergehalts und/oder einer Sauerstoffsättigung, welche beispielsweise im entsprechenden Gewebe festgestellt werden.

In einer weiteren Ausführungsform wird der Betrachtungsbereich mit einer Lichtquelle beleuchtet, wobei die Lichtquelle insbesondere zum Beleuchten des Betrachtungsbereichs mit einem zu einer spektralen Auswertung, insbesondere mittels einer HSI oder MSI, korrespondierenden Lichtspektrum eingerichtet ist.

Um auch bei fehlendem Vorliegen einer entsprechenden Referenzspektralverteilung zu einer speziellen rotatorischen Position und/odereinem speziellen Abstand einen zuverlässigen Betrieb der medizinischen Bildgebungsvorrichtung sicherzustellen, und/oder lediglich eine ökonomische Anzahl von Referenzspektralverteilungen im Fertigungsprozess ermitteln zu müssen, wird die Referenzspektralverteilung mittels einer Interpolation aus Referenzspektralverteilungen, insbesondere gespeicherten Referenzspektralverteilungen eines bekannten Abstands oder bekannter Abstände und/oder einer bekannten rotatorischen Position oder bekannten rotatorischen Positionen ermittelt.

So kann beispielsweise bei Vorliegen einer Referenzspektralverteilung linksseitig der aktuell vorliegenden rotatorischen Position und Vorliegen einer weiteren Referenzspektralverteilung rechtsseitig der aktuell vorliegenden rotatorischen Position mittels der Interpolation ein entsprechender Zwischenbereich die aktuell vorliegende rotatorische Position abgedeckt werden und ein Kalibrieren trotz fehlender Referenzspektralverteilung für diese aktuell vorliegende rotatorische Position durchgeführt werden.

Eine "Interpolation" beschreibt dabei ein mathematisches Vorgehen, bei welchem zu vorliegenden diskreten Daten, beispielsweise Messwerten, eine stetige Funktion gefunden wird, welche auch Zwischenbereiche der vorliegenden diskreten Daten abbilden. Die zwischen den diskreten Daten vorliegenden Werte werden daher im Sinne einer Approximation ermittelt und ergänzt, sodass entsprechend unbekannte Zwischenwerte möglichst präzise ermittelt werden.

In einem weiteren Aspekt wird die Aufgabe gelöst durch eine medizinische Bildgebungsvorrichtung, insbesondere ein medizinisches Endoskop-System oder ein medizinisches Exoskop-System, welches zum Durchführen eines Verfahrens gemäß einer oder mehrerer der vorig beschriebenen Ausführungsformen eingerichtet ist.

Eine solche medizinische Bildgebungsvorrichtung kann zuverlässig und genau Bilder und/oder Bildinformationen des Betrachtungsbereiches ermitteln, auch wenn beispielsweise ein optisches System und/oder eine Optik der medizinischen Bildgebungsvorrichtung einer Rotation unterliegt und/oder ein Abstand des jeweiligen optischen Systems und/oder der jeweiligen Optik zum Betrachtungsbereich verändert wird.

Beispielsweise ist ein solches Endoskop-System ein optisches Endoskop mit einem Okular sowie einer auf das Okular aufgesetzten Kamera mit beispielsweise einem Drehwinkelsensor zum Bestimmen einer rotatorischen Position der Kamera in Bezug zum optischen System des optischen Endoskops.

Im Weiteren wird die Erfindung anhand von Ausführungsbeispielen näher erläutert. Es zeigen
- Figur 1: eine Untersuchungssituation mit einem Endoskop in schematischer Seitenansicht,
- Figur 2 a) bis c): eine schematische Darstellung der Drehung eines Bildes mit mathematischer Winkeldarstellung,

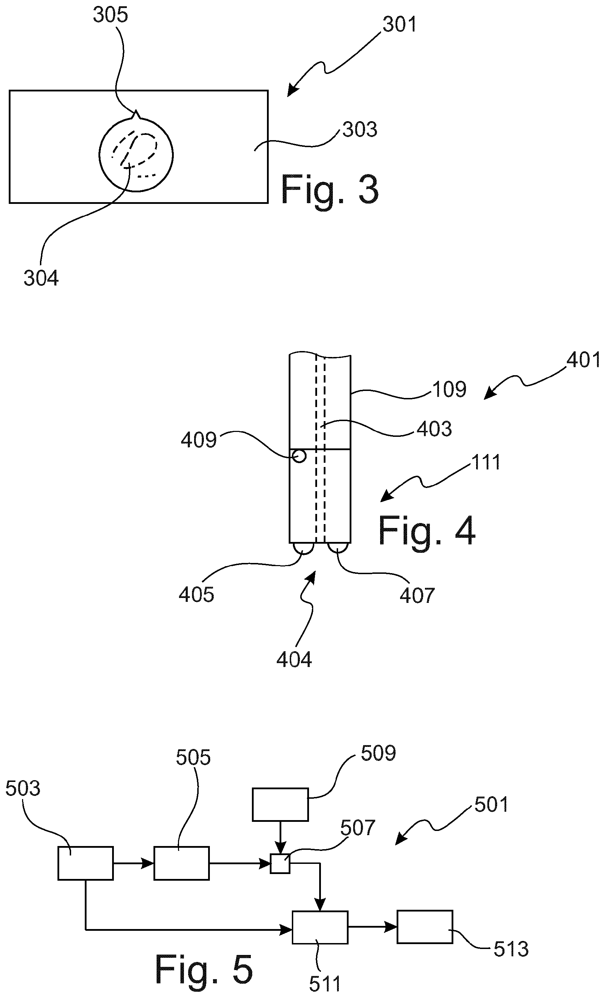

- Figur 3: einen Sichtausschnitt mit einem entsprechenden Bild des Endoskopes der Figur 1,
- Figur 4: eine Vergrößerung der Spitze des Endoskopes der Figur 1 in einer schematischen Seitenansicht, sowie
- Figur 5: ein Verfahren zum Kalibrieren entsprechender Bilder.

Eine Untersuchungssituation 101 zeigt ein Endoskop 103. Das Endoskop 103 weist eine Griff 105 zum Fassen und Bedienen des Endoskops 103 durch einen Bediener sowie ein Okular 107 zum Einblick für den Bediener auf. Auf das Endoskop 103 ist am Okular 107 eine Kamera 108 aufgesetzt, sodass ein eigentlich im Okular 107 für den Bediener sichtbar aufbereitete Bild mittels der Kamera 108 aufgenommen wird. Weiterhin weist das Endoskop 103 einen Schaft 109 mit einer Spitze 111 auf. Mittels des Endoskops 103 wird innerhalb einer Bauchdecke 121 ein Organ 123 betrachtet und ein Bild des Organs 123 erzeugt und von der Kamera 108 aus dem Okular 107 aufgenommen. Die Spitze 111 des Endoskops 103 ist dabei in einem Abstand 131 vom Organ 123 angeordnet. Ein Drehsensor 106, welcher ein optischer Inkrementgeber ist, ermittelt eine Verdrehung der Kamera 108 gegenüber dem Okular 107.

Das Endoskop 103 weist weiterhin Einrichtungen zum Beleuchten des Organs 123 auf, sodass das Organ sichtbar ist und als Nebeneffekt innerhalb der Bauchdecke 121 auch Reflexionen 141 entstehen. Diese Reflexionen 141 verfälschen das vom Endoskop 103 aufgenommene und dem Betrachter zugeführte Bild.

Weiterhin weist das Endoskop 103 eine Auswerteeinheit 161 auf, auf welcher sowohl das mit der Kamera 108 aufgenommene Bild des Organs 123 sowie zusätzliche Informationen angezeigt werden. So kann mittels der Auswerteeinheit 161 neben dem Bild des Organs 123 ein überlagertes Bild einer Multispektral-Imaging-(MSI)-Aufnahme dargestellt werden, entsprechend können Durchblutungs- oder auch Sauerstoffsättigungswerte des Organs 123 angezeigt werden.

Ein Bild 201 stellt eine entsprechende Aufnahme des Organs 123 dar. Das Bild 201 weist einen kreisförmigen Rand 203 sowie einem Nullradius 251 zugeordnet einen Indikator 205 auf. Das Bild 201 weist weiterhin eine Drehachse 211 auf. Beispielhaft sind hier eine Bildfläche 207 sowie eine Bildfläche 209 gezeigt, welche spektrale Verfälschungen durch die Reflexionen 141 aufweisen.

Wird das Bild 201 von der Ausrichtung auf den Nullradius 251 auf den Drehradius 252 um einen Drehwinkel 253 verdreht, so wandern auch die beispielhaft gezeigte Bildflächen 207 und 209 rotatorisch mit dem Bild 201. Weiterhin kann es zu einer Verschiebung der Drehachse 211 im Bild 201 durch eine Präzession kommen. Die Ursache hierfür ist beispielsweise ein nicht mittiges Anordnen einer entsprechenden Drehachse der Kamera 108 gegenüber dem Okular 107.

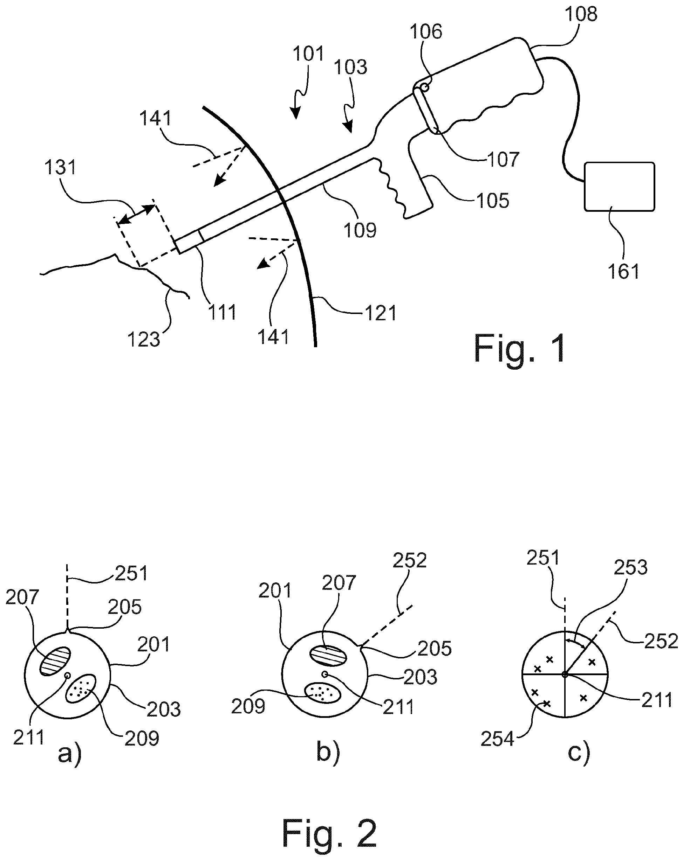

Wird das Bild 201 von der Ausrichtung auf den Nullradius 251 auf eine Ausrichtung zum Drehradius 252 um den Drehwinkel 253 verdreht, so wandern auch entsprechend beispielhaft dargestellte Referenzpunkte 254 im Bild 201 mit (vergleiche Figur 2c)). Anhand dieser Referenzpunkte, welche beispielsweise signifikante Farbunterschiede oder Kontrastunterschiede abbilden, wird mittels einer Bildauswertung der Drehwinkel 253 bestimmt.

In einer Alternativen kann anhand einer Bildauswertung des Indikators 205 der Drehwinkel 253 bestimmt werden, dies kann auch redundant zum Bestimmen des Drehwinkels 253 mittels der Referenzpunkte 254 erfolgen. In einer weiteren Alternativen, welche auch redundant mit der Bildauswertung und/oder der Bildauswertung des Indikators 205 eingesetzt werden kann, wird der Drehwinkel 253 mittels des Drehsensors 106 ermittelt.

Ein Sichtausschnitt 301 mit einem Hintergrund 303 zeigt eine mögliche Darstellung auf der Auswerteeinheit 161. Beispielhaft ist hier ein Bild 304 gezeigt, welches sowohl ein optisches Bild sowie auch überlagerte farbliche Informationen bezüglich eines Multispektral-Imaging enthalten. Eine Ecke 305 des Bildes 304 dient dabei als Indikator für eine rotatorische Position, womit analog zum Drehwinkel 253 ein Drehwinkel des Bildes 304 bestimmbar ist.

Eine Vergrößerung 401 des Endoskops 103 zeigt neben einem Teil des Schaftes 109 sowie der Spitze 111 einen Bildleiter 403. Mittels des Bildleiters 403 wird das Bild des Organs 123 weitergeleitet und im Endoskop 103 in ein sichtbares Bild umgewandelt. Dieses sichtbare Bild kann dann mittels des Okulars 107 und der Kamera 108 auf der Auswerteeinheit 161 angezeigt werden. Weiterhin ist an einem Endbereich 404 der Spitze 111 eine Beleuchtung 405 angeordnet. Die Beleuchtung 405 ist dabei eine multispektrale Beleuchtung, sodass einzelne Spektralbereiche gezielt zum Beleuchten de Organs 123 erzeugt werden können. Damit ist das Feststellen von Gewebeeigenschaften mittels einer Spektralauswertung möglich.

Weiterhin weist die Spitze 111 einen Lasersensor 407 zum Bestimmen des Abstandes 131 auf.

In einer Alternativen kann auch die Spitze eines jeweiligen Endoskopes 103 oder Exoskopes drehbar sein, beispielsweise wenn statt der Kamera 108 ein integrierter Bildaufnehmer im Endoskop verwendet wird. Ein Drehsensor 409 tastet dabei einen Inkrementring (nicht gezeigt) am Schaft 109 des Endoskopes 103 ab. Mittels des Drehsensors 409 kann alternativ oder redundant zur beschriebenen Bildauswertung der Drehwinkel 253 bestimmt werden.

Folglich ist in der Untersuchungssituation 101 sowohl der Abstand 131 zum Organ 123 als auch der Drehwinkel 253, abhängig von der Kamera 108 oder alternativ oder zusätzlich von der Spitze 111, am Endoskop 103 zuverlässig und genau ermittelbar.

Ein entsprechendes Verfahren 501 zum Kalibrieren des Endoskops 103 sei detailliert wie folgt beschrieben:

Es erfolgt ein Aufnehmen 503 eines Bildes des Organes 123 in dem Abstand 131 mit dem Drehwinkel 253. Damit liegt ein verfälschtes Bild des Organes 123 vor. Es erfolgt dann ein Bestimmen 505 der Spektralverteilung dieser aufgenommenen Bildinformation, sodass die Spektralverteilung für die weitere Auswertung vorliegt. Diese Spektralverteilung wird einem Ermitteln 507 einer Spektralabweichung zugeführt. Das Ermitteln 507 greift dabei auf eine Referenzspektralverteilung 509 zurück, sodass mittels einer Differenzbildung eine Abweichung von dieser Referenzspektralverteilung ermittelt ist. Die Abweichung ist dabei die Abweichung der bestimmten Spektralverteilung von der Referenzspektralverteilung.

Sodann erfolgt mittels eines Überlagerns der Abweichung von der Referenzspektralverteilung mit der ursprünglich aufgenommenen Bildinformation derart, dass eine korrigierte Bildinformation 513 erzeugt wird. Die korrigierte Bildinformation 513 enthält dabei die aufgenommene Bildinformation, wobei die Spektralverteilung derart angepasst ist, dass diese der realen Spektralverteilung der Bildinformation des Organs 123 entspricht. Somit kann anhand dieser kalibrierten Messung mit der korrigierten Bildinformation eine exakte Auswertung der Bildinformation und Darstellung auf der Auswerteeinheit 161 erfolgen.

### Bezugszeichenliste

- 101: Untersuchungssituation
- 103: Endoskop
- 105: Griff
- 107: Okular
- 108: Kamera
- 109: Schaft
- 111: Spitze
- 121: Bauchdecke
- 123: Organ
- 131: Abstand
- 141: Reflexion
- 161: Auswerteeinheit
- 201: Bild
- 203: Rand
- 205: Indikator
- 207: Bildfläche
- 209: Bildfläche
- 211: Drehachse
- 251: Nullradius
- 252: Drehradius
- 253: Drehwinkel
- 254: Referenzpunkte
- 301: Sichtausschnitt
- 303: Hintergrund
- 304: Bild
- 305: Ecke
- 401: Vergrößerung
- 403: Bildleiter
- 404: Endbereich
- 405: Beleuchtung
- 407: Lasersensor
- 409: Drehsensor
- 501: Verfahren zum Kalibrieren
- 503: Aufnehmen
- 505: Bestimmen
- 507: Ermitteln
- 509: Referenzspektralverteilung
- 511: Korrigieren
- 513: korrigierte Bildinformation

## Patentansprüche

1. Verfahren zum Kalibrieren einer medizinischen Bildgebungsvorrichtung (103, 108, 161), insbesondere eines Endoskop-Systems oder eines Exoskop-Systems, wobei die medizinische Bildgebungsvorrichtung (103) ein optisches System (111) mit einer Optik (403) und einem Bildsensor (108) zum Aufnehmen eines Bildes (301) eines Betrachtungsbereiches (123) aufweist und eine Bildinformation des Bildes durch einen inneren und/oder äußeren Einfluss, insbesondere durch das optische System (111, 108), durch eine Beleuchtung (405) und/oder durch eine Umgebungsbedingung (131), einer Spektralabweichung unterliegt, mit folgenden Schritten:
- Aufnehmen (503) einer Bildinformation eines Bildbereiches, so dass eine erste Aufnahme vorliegt,
- Bestimmen (505) einer Spektralverteilung der ersten Aufnahme, sodass eine Spektralverteilung der ersten Aufnahme vorliegt,
- Ermitteln (507) einer Spektralabweichung anhand der Spektralverteilung der ersten Aufnahme und einer Referenzspektralverteilung (509), sodass eine Abweichung von der Referenzspektralverteilung vorliegt,
- Korrigieren (511) der Bildinformation auf Basis der Abweichung von der Referenzspektralverteilung (509), sodass eine korrigierte Bildinformation vorliegt,
sodass die Bildinformation derart aufbereitet ist, dass die korrigierte Bildinformation mit einer reduzierten oder eliminierten Spektralabweichung vorliegt,
wobei das Korrigieren der Bildinformation anhand einer ermittelten rotatorischen Position des optischen Systems und/oder der Optik durchgeführt wird, und/oder
wobei ein Abstand (131) vom Betrachtungsbereich ermittelt und die Referenzspektralverteilung (509) abhängig von diesem Abstand (131) von dem Betrachtungsbereich bestimmt ist, wobei das Korrigieren der Bildinformation anhand des ermittelten Abstands (131) vom Betrachtungsbereich durchgeführt wird..

2. Verfahren gemäß Anspruch 1, **dadurch gekennzeichnet, dass** das Aufnehmen einer Bildinformation, das Bestimmen einer Spektralverteilung, das Ermitteln einer Spektralabweichung und/oder das Korrigieren der Bildinformation für ein Pixel oder für ein jeweiliges Pixel des Bildes und/oder der Bildinformation durchgeführt wird.

3. Verfahren gemäß Anspruch 1 oder 2, **dadurch gekennzeichnet, dass** das Aufnehmen einer Bildinformation, das Bestimmen einer Spektralverteilung, das Ermitteln einer Spektralabweichung und/oder das Korrigieren der Bildinformation für einen Spektralbereich oder mehrere Spektralbereiche erfolgt.

4. Verfahren gemäß Anspruch 3, **dadurch gekennzeichnet, dass** ein Korrigieren der Bildinformation auf Basis der Abweichung von der Referenzspektralverteilung (509) für einen Spektralbereich oder mehrere Spektralbereiche erfolgt, sodass die Bildinformation derart aufbereitet ist, dass die korrigierte Bildinformation mit einer reduzierten oder eliminierten Spektralabweichung für einen jeweiligen Spektralbereich oder mehrere jeweilige Spektralbereiche vorliegt.

5. Verfahren gemäß einem der vorherigen Ansprüche, **dadurch gekennzeichnet, dass** der ermittelte Abstand mittels eines Lasers (407), eines Ultraschallsystems und/oder mittels einer Bildauswertung bestimmt wird.

6. Verfahren gemäß einem der vorherigen Ansprüche, **dadurch gekennzeichnet, dass** die rotatorische Position anhand eines dem optischen System und/oder der Optik zugeordneten optischen Indikators (205, 305) für die rotatorische Position ermittelt wird.

7. Verfahren gemäß Anspruch 6, **dadurch gekennzeichnet, dass** die rotatorische Position mittels einer Bildauswertung zum Erkennen des optischen Indikators (205, 305) für die rotatorische Position ermittelt wird.

8. Verfahren gemäß einem der vorherigen Ansprüche, **dadurch gekennzeichnet, dass** das die rotatorische Position anhand eines Sensors (106, 409) ermittelt wird.

9. Verfahren gemäß Anspruch 8, **dadurch gekennzeichnet, dass** die rotatorische Position anhand eines Magnet-Sensors, eines Hall-Sensors, eines Laser-Sensors, eines Lichtsensors und/oder eines Inkrementgebers ermittelt wird.

10. Verfahren gemäß einem der vorherigen Ansprüche, **dadurch gekennzeichnet, dass** die korrigierte Bildinformation für ein HSI oder ein MSI ausgewertet wird, insbesondere zum Bestimmen von Eigenschaften des Betrachtungsbereichs, insbesondere eines Hämoglobingehalts, eines Wassergehaltes und/oder einer Sauerstoffsättigung.

11. Verfahren gemäß einem der vorherigen Ansprüche, **dadurch gekennzeichnet, dass** der Betrachtungsbereich (123) mit einer Lichtquelle (405) beleuchtet wird, wobei die Lichtquelle (405) insbesondere zum Beleuchten des Betrachtungsbereichs (123) mit einem zu einer spektralen Auswertung, insbesondere mittels einer HSI oder einer MSI, korrespondierenden Lichtspektrum, eingerichtet ist.

12. Verfahren gemäß einem der vorherigen Ansprüche, **dadurch gekennzeichnet, dass** die Referenzspektralverteilung (509) mittels einer Interpolation aus Referenzspektralverteilungen, insbesondere gespeicherten Referenzspektralverteilungen eines bekannten Abstands oder bekannter Abstände und/oder einer bekannten rotatorischen Position oder bekannten rotatorischen Positionen ermittelt wird.

13. Medizinische Bildgebungsvorrichtung, insbesondere medizinisches Endoskop-System (103, 108, 161) oder medizinisches Exoskop-System, welches zum Durchführen eines Verfahrens gemäß einem der Ansprüche 1 bis 12 eingerichtet ist.

## Claims

1. Method for calibrating a medical imaging device (103, 108, 161), in particular an endoscope system or an exoscope system, wherein the medical imaging device (103) has an optical system (111) which has an optical unit (403) and an image sensor (108) for capturing an image (301) of a viewing region (123), and image information regarding the image is subject to a spectral deviation due to an internal and/or external influence, in particular due to the optical system (111, 108), due to an illumination (405) and/or due to an environmental condition (131), the method comprising the following steps:
- recording (503) image information regarding an image region so that a first recording is obtained,
- identifying (505) a spectral distribution of the first recording so that a spectral distribution of the first recording is obtained,
- determining (507) a spectral deviation based on the spectral distribution of the first recording and a reference spectral distribution (509) so that a deviation from the reference spectral distribution is obtained,
- correcting (511) the image information on the basis of the deviation from the reference spectral distribution (509) so that corrected image information is obtained,
so that the image information is processed such that the corrected image information is obtained with a reduced or eliminated spectral deviation,
wherein the correction of the image information is carried out based on a determined rotational position of the optical system and/or the optical unit, and/or
wherein a distance (131) from the viewing region is determined and the reference spectral distribution (509) is identified depending on this distance (131) from the viewing region, wherein the correction of the image information is carried out based on the determined distance (131) from the viewing region.

2. Method according to claim 1, **characterized in that** the recording of image information, the identification of a spectral distribution, the determination of a spectral deviation and/or the correction of the image information is carried out for a pixel or for a relevant pixel of the image and/or of the image information.

3. Method according to claim 1 or 2, **characterized in that** the recording of image information, the identification of a spectral distribution, the determination of a spectral deviation and/or the correction of the image information is carried out for one spectral region or a plurality of spectral regions.

4. Method according to claim 3, **characterized in that** a correction of the image information is carried out on the basis of the deviation from the reference spectral distribution (509) for one spectral region or a plurality of spectral regions so that the image information is processed such that the corrected image information is obtained with a reduced or eliminated spectral deviation for one relevant spectral region or a plurality of respective spectral regions.

5. Method according to any of the preceding claims, **characterized in that** the determined distance is identified by means of a laser (407), an ultrasound system and/or by means of image analysis.

6. Method according to any of the preceding claims, **characterized in that** the rotational position is determined using an optical indicator (205, 305) for the rotational position, which is assigned to the optical system and/or the optical unit.

7. Method according to claim 6, **characterized in that** the rotational position is determined by means of image evaluation to detect the optical indicator (205, 305) for the rotational position.

8. Method according to any of the preceding claims, **characterized in that** the rotational position is determined using a sensor (106, 409).

9. Method according to claim 8, **characterized in that** the rotational position is determined using a magnetic sensor, a Hall sensor, a laser sensor, a light sensor and/or an incremental encoder.

10. Method according to any of the preceding claims, **characterized in that** the corrected image information is evaluated for an HSI or an MSI, in particular for identifying properties of the viewing region, in particular a hemoglobin content, a water content and/or an oxygen saturation.

11. Method according to any of the preceding claims, **characterized in that** the viewing region (123) is illuminated using a light source (405), the light source (405) being configured in particular to illuminate the viewing region (123) using a light spectrum corresponding to a spectral evaluation, in particular by means of an HSI or an MSI.

12. Method according to any of the preceding claims, **characterized in that** the reference spectral distribution (509) is determined by means of an interpolation from reference spectral distributions, in particular stored reference spectral distributions of a known distance or known distances and/or a known rotational position or known rotational positions.

13. Medical imaging device, in particular a medical endoscope system (103, 108, 161) or medical exoscope system, which is configured to carry out a method according to any of claims 1 to 12.

## Revendications

1. Procédé pour le calibrage d'un dispositif d'imagerie médicale (103, 108, 161), en particulier d'un système d'endoscope ou d'un système d'exoscope, dans lequel le dispositif d'imagerie médicale (103) présente un système optique (111) comportant une optique (403) et un capteur d'image (108) permettant d'enregistrer une image (301) d'une zone d'observation (123) et des informations d'image de l'image sont soumises à une déviation spectrale par une influence interne et/ou externe, en particulier par le système optique (111, 108), par un éclairage (405) et/ou par une condition d'environnement (131), comportant les étapes suivantes :
- enregistrement (503) d'informations d'image d'une zone d'image, de sorte qu'il existe un premier enregistrement,
- définition (505) d'une distribution spectrale du premier enregistrement, de sorte qu'il existe une distribution spectrale du premier enregistrement,
- détermination (507) d'un écart spectral à l'aide de la distribution spectrale du premier enregistrement et d'une distribution spectrale de référence (509), de sorte qu'il existe un écart par rapport à la distribution spectrale de référence,
- correction (511) des informations d'image sur la base de l'écart par rapport à la distribution spectrale de référence (509), de sorte qu'il existe des informations d'image corrigées,
de sorte que les informations d'image sont traitées de telle sorte que les informations d'image corrigées existent avec un écart spectral réduit ou éliminé,
dans lequel la correction des informations d'image est effectuée à l'aide d'une position de rotation déterminée du système optique et/ou de l'optique, et/ou
dans lequel une distance (131) par rapport à la zone d'observation est déterminée et la distribution spectrale de référence (509) est définie en fonction de ladite distance (131) par rapport à la zone d'observation, dans lequel la correction des informations d'image est effectuée à l'aide de la distance (131) déterminée par rapport à la zone d'observation.

2. Procédé selon la revendication 1, **caractérisé en ce que** l'enregistrement d'informations d'image, la définition d'une distribution spectrale, la détermination d'un écart spectral et/ou la correction des informations d'image sont effectués pour un pixel ou pour un pixel respectif de l'image et/ou des informations d'image.

3. Procédé selon la revendication 1 ou 2, **caractérisé en ce que** l'enregistrement d'informations d'image, la définition d'une distribution spectrale, la détermination d'un écart spectral et/ou la correction des informations d'image sont effectués pour un domaine spectral ou pour plusieurs domaines spectraux.

4. Procédé selon la revendication 3, **caractérisé en ce qu'**une correction des informations d'image est effectuée sur la base de l'écart par rapport à la distribution spectrale de référence (509) pour un domaine spectral ou pour plusieurs domaines spectraux, de sorte que les informations d'image sont traitées de telle sorte que les informations d'image corrigées existent avec un écart spectral réduit ou éliminé pour un domaine spectral respectif ou pour plusieurs domaines spectraux respectifs.

5. Procédé selon l'une des revendications précédentes,
**caractérisé en ce que** la distance déterminée est définie au moyen d'un laser (407), d'un système à ultrasons et/ou au moyen d'une évaluation d'image.

6. Procédé selon l'une des revendications précédentes,
**caractérisé en ce que** la position de rotation est déterminée à l'aide d'un indicateur optique (205, 305) pour la position de rotation associé au système optique et/ou à l'optique.

7. Procédé selon la revendication 6, **caractérisé en ce que** la position de rotation est déterminée au moyen d'une évaluation d'image pour la reconnaissance de l'indicateur optique (205, 305) pour la position de rotation.

8. Procédé selon l'une des revendications précédentes,
**caractérisé en ce que** la position de rotation est déterminée à l'aide d'un capteur (106, 409).

9. Procédé selon la revendication 8, **caractérisé en ce que** la position de rotation est déterminée à l'aide d'un capteur magnétique, d'un capteur à effet Hall, d'un capteur laser, d'un capteur de lumière et/ou d'un capteur incrémental.

10. Procédé selon l'une des revendications précédentes,
**caractérisé en ce que** les informations d'image corrigées sont évaluées pour un système d'imagerie hypersectrale ou un système d'imagerie multispectrale, en particulier pour la définition de propriétés de la zone d'observation, en particulier d'une teneur en hémoglobine, d'une teneur en eau et/ou d'une saturation en oxygène.

11. Procédé selon l'une des revendications précédentes,
**caractérisé en ce que** la zone d'observation (123) est éclairée par une source lumineuse (405), dans lequel la source lumineuse (405) est en particulier configurée pour éclairer la zone d'observation (123) avec un spectre lumineux correspondant à une évaluation spectrale, en particulier au moyen d'un système d'imagerie hypersectrale ou d'un système d'imagerie multispectrale.

12. Procédé selon l'une des revendications précédentes,
**caractérisé en ce que** la distribution spectrale de référence (509) est déterminée au moyen d'une interpolation à partir de distributions spectrales de référence, en particulier de distributions spectrales de référence mémorisées d'une distance connue ou de distances connues et/ou d'une position de rotation connue ou de positions de rotation connues.

13. Dispositif d'imagerie médicale, en particulier système d'endoscope médical (103, 108, 161) ou système d'exoscope médical, lequel est configuré pour mettre en œuvre un procédé selon l'une des revendications 1 à 12.
